Europäisches Patentamt

European Patent Office

Office européen des brevets

(11) Veröffentlichungsnummer : **0 640 933 A2**

## (12) EUROPÄISCHE PATENTANMELDUNG

(21) Anmeldenummer : **94113143.5**

(51) Int. Cl.⁶ : **G06K 9/20, A61B 5/117**

(22) Anmeldetag : **23.08.94**

(30) Priorität : **23.08.93 DE 4328638**

(43) Veröffentlichungstag der Anmeldung :
**01.03.95 Patentblatt 95/09**

(84) Benannte Vertragsstaaten :
**AT BE CH DE DK ES FR GB GR IE IT LI NL SE**

(71) Anmelder : **GIM-Gesellschaft für Innovation
und Management mbH
An der Salza 8a
D-99734 Nordhausen (DE)**

(72) Erfinder : **Keitz,Carsten
Jacob-Plaut-Strasse 8
D-99734 Nordhausen (DE)**
Erfinder : **Kösters,Heiner Dr.
Chopin-Strasse
D-99734 Nordhausen (DE)**
Erfinder : **Franke,Jürgen
Am Rossmannsbach 22
D-99734 Nordhausen (DE)**
Erfinder : **Piehler,Gerd
Friedeweg 125
D-99759 Sollstedt (DE)**

(54) **Verfahren und Anordnung zur Mustererkennung.**

(57) Bei bekannten Fingerabdruckaufnahmevorrichtungen wird der Start der Bildaufnahme durch aufwendige softwaretechnische Lösungen realisiert. Auch die Täuschung durch aufgelegte Fingerabdruckfotografien wird nicht verhindert. In der vorgestellten Fingerabdruckaufnahmevorrichtung wird der Aufnahmestart durch eine Fingerauflagedrucküberwachung realisiert. Die Täuschung durch Fotografien wird durch eine Veränderung im optischen System verhindert.

Eine tatsächliche Identifizierung erfolgt über die Erfassung der Disloziierung eindeutiger subjektiver Merkmale.

Die Fingerabdruckaufnahmevorrichtung (2) wird beweglich angeordnet und mit einem elastischen Element (10) in der Ruhelage gehalten. Der mit dem elastischen Element (10) gekoppelte Drucksensor (11) erzeugt das Aufnahmestartsignal. Die Täuschung durch Fotografien wird durch eine stärkere Kippung des Prismas (7) verhindert.

Die Fingerabdruckaufnahmevorrichtung wird in Hochsicherheitebereichen eingesetzt, um nur zugelassenen Personen den Zugang zu Räumen und die Nutzung von speziellen Geräten zu ermöglichen.

EP 0 640 933 A2

Die Erfindung betrifft ein Verfahren und eine Anordnung zur Mustererkennung, insbesondere Fingerabdruckidentifikation, durch Auflage des Musters auf eine Bildaufnahmevorrichtung zur optischen Aufbereitung des zu erkennenden Musterbildes sowie deren Bearbeitung und Erkennung über eine elektronische Verarbeitungseinheit.

Ein Fingerabdruck-Identifikationssystem wird beispielsweise als Zugangskontrolle für Sicherheitsbereiche eingesetzt. Bekannt sind DE 4220971AI sowie DE 3712089 A1.

Dabei basiert die technische Lösung auf dem Prinzip der Merkmalerkennung nach der zweidimensionalen Fourier-Transformation.

Es werden regelmäßig die Endabschnitte, Linien sowie Anzahl (Merkmale) des Fingerabdrucks herangezogen.

So auch nach DE 3712089AI, wobei die Identifikation des Papillarlinienmusters mit einem ein segmentales Bildsignal erzeugenden lichtempfindlichen Teil und ein Vergleichen des Bildsignals mit Vorgabesignalen erfolgt.

Bekannt ist ferner EP 0308162 zu Verfahren und Anordnung zur Erzeugung und Auswertung von Fingerabdruck-Abbildern. Das optische System ist dabei gekennzeichnet durch die Bildaufnahmevorrichtung mit Beleuchtung, Prisma, Objektivlinse, Korrekturprisma, Blende zur Vermeidung der Aberration und Reihendetektor als CCD-Element. Es erfolgt eine Musterauflageerkennung zum Starten des Erkennungsprozesses, insbesondere durch einen Druckschalter.

DE 3006677 beschreibt eine Einrichtung zur Aufnahme von Fingerabdrücken unter Verwendung einer Wiedergabefläche zur Aufnahme der Fingerabdrücke und einer zugeordneten Auswertestation mit einer Bildübertragungseinrichtung, beispielsweise optischen Abbildungseinrichtung mit zugeordneter Fernsehaufnahmeröhre. Zum daktyloskopischen Vergleich wird die Umsetzung der Merkmale des Fingerabdruckes in alphanumerische Zeichen, die in einer Vergleichseinrichtung mit gespeicherten Zeichenfolgen verglichen werden beschrieben oder es wird auf optischem Weg ein Abdruck mit einem gespeicherten Vergleichsabdruck verglichen.

Bekannt ist ferner DE 4016832, eine Türverschlußeinrichtung, insbesondere für Kraftfahrzeugtüren, die einen Sensor aufweist zur Erfassung wenigstens eines Fingerabdruckes sowie einem Speicher für Referenzinformation und einer Vergleichseinrichtung, die bei erkannter Übereinstimmung eine Vorrichtung entriegelt oder verriegelt.

Alle bekannten Darstellungen und Systeme weisen jedoch wesentliche Mängel auf. So sind selbige nicht in der Lage, einen nachgemachten Abdruck (bspw. Folie) von einem Lebenden zu unterscheiden. Ferner sind gewählte Anordnungen sehr aufwendig, um die optischen Probleme bei Bildaufnahme, wie gleichmäßig scharfe Abbildung auf der Sensorfläche, Aberration, Muster liegt nicht gleichmäßig bzw. exakt auf der definierten Auflagefläche, zu beherrschen.

Es bestand daher die Aufgabe ein System zu entwickeln, das die dargestellten Nachteile überwindet.

Darüber hinaus sollte die Identifizierung über die Erfassung der Dislozierung eindeutiger subjektiver Merkmale erfolgen, um eine exakte sowie tatsächliche Identifizierung des lebenden Idividuums zu ermöglichen.

So soll eine Anordnung zum fehlertoleranten Mustervergleich, insbesondere durch unterschiedliche Positionierung des Musters auf der Bildaufnahmevorrichtung, entstehen.

Die Aufgabe wird erfindungsgemäß durch die in den Ansprüchen 1 bis 6 gekennzeichneten Merkmale gelöst.

Die Mustererkennung, insbesondere Fingerabdruck-Identifikation erfolgt durch Auflage des Musters auf eine Bildaufnahmevorrichtung zur optischen Aufbereitung des zu erkennenden Musters. Diese Bildaufnahmevorrichtung besteht aus einem Dreikantenprisma, von dem eine Fläche als Musterauflagefläche dient, einer Lichtquelle, einer Objektivlinse bzw. Linsensystem und einem optischen Sensor, insbesondere CCD-Sensor.

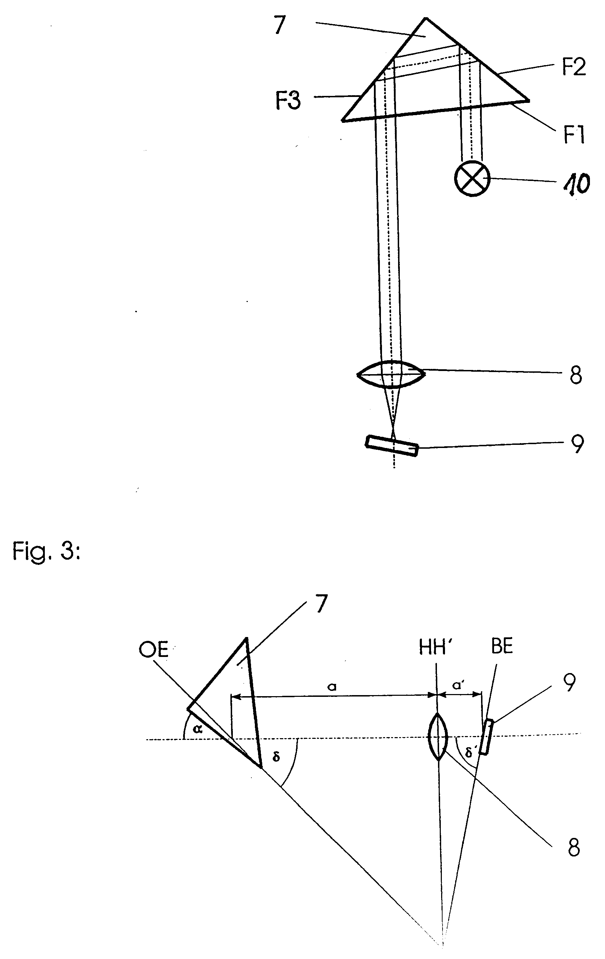

Vorteilhaft ist, daß erfindungsgemäß die Musterauflagefläche des Dreikantenprismas in einem Winkel alpha = 25 bis 44° (Fig.3) zur optischen Achse der Objektivlinse angeordnet ist, um Täuschungen des Systems durch aufgelegte Bilder oder Folien zu verhindern.

Ferner wird durch die Auflage eines Musters, insbesondere Fingers, die Totalreflexion an der Prismenfläche gestört. Dabei ist von besonderem Vorteil, daß durch die veränderten Reflexionseigenschaften ein kontrastreiches Musterabdruckbild entsteht, vornehmlich ein Fingerabdruckbild.

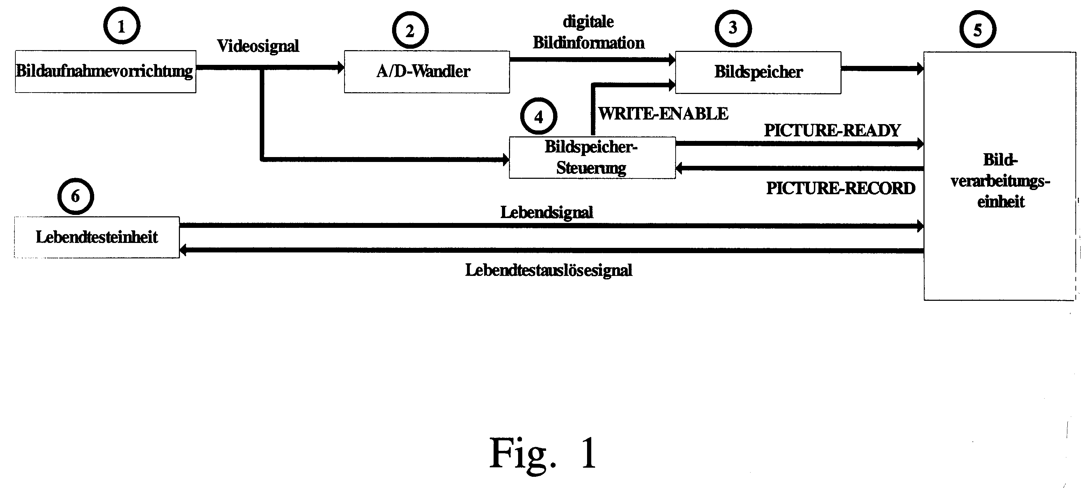

Die Anordnung zur Mustererkennung besteht nach Figur 1 aus Bildaufnahmevorrichtung (1), Analog/Digital-Wandler (2), Bildspeicher (3), Bildspeichersteuerung (4), Bildverarbeitungseinheit (5) und Lebendtest (6).

Die Bildaufnahmevorrichtung (1) besteht aus Dreikantprisma (7), Objektivlinse (8), CCD-Sensor (9) und Lichtquelle (10). (siehe Figur 3)

Die von der Lichtquelle (10) emittierten Lichtstrahlen werden über die Fläche (F1) in das Prisma eingekoppelt, an den Flächen (F2) und (F3) totalreflektiert und über die Objektivlinse (8) auf den CCD-Sensor (9) projiziert. Wird ein Finger auf die Prismafläche (F3) aufgelegt, verändern sich die Reflexionseigenschaften der Prismafläche (F3). Dies bedeutet, daß die Totalreflexion an der Prismafläche (F3) gestört wird. Durch die veränderten Reflexionseigenschaften entsteht ein kontrastreiches Fingerabdruckbild. Die Bergabschnitte der Fingerabdrucklinien, die die Prismafläche (F3) berühren, erzeugen dunkle Linien. Die Talabschnitte des Fingerabdruckes werden durch helle Linien dargestellt, die Totalreflexion wird an diesen Stellen nicht so stark beeinflußt. Durch eine optimierte Anordnung der optischen Bauteile der Bildaufnahmevorrichtung (1) nach Fig. 3 wird auf dem CCD-Sensor (9) ein gleichmäßig scharfes Bild erzeugt.

Der CCD-Sensor (9) wird unter Einhaltung der Scheimpflug-Bedingung in seiner Abbildungsebene (BE) zur optischen Achse (OA) gekippt (Winkel delta).

$$tan(delta')/tan(delta) = a/a'$$

Dabei tritt Winkel delta zwischen optischer Achse (OA) und Hilfslinie (OE) auf. Durch die Kippung entsteht auf der CCD-Sensor (9) ein verzerrtes Bild. Die Verzerrung ist durch eine mathematische Beziehung beschreibbar und wird rechentechnisch aufgehoben. Steht die Prismafläche (F3) im Winkel alpha=45° zur optischen Achse (OA) der Objektivlinse, kann das Erkennungssystem mit einer aufgelegten Fingerabdruckfotografie getäuscht werden. Zur Verhinderung der Täuschung wird die Prismafläche (F3) in einem Winkel alpha = 25 bis 44° angeordnet. Unter Einhaltung dieses Winkels kann eine aufgelegte Fingerabdruckfotografie nicht mehr erkannt werden.

Erfindungsgemäß wird vorgeschlagen,die Fingerauflage im Winkel von 15 Grad zur Gehäusegrundfläche anzuordnen.Dadurch ist eine vorteilhafte Auflage gegeben.

Das Aufnahmemodul besteht vorteilhaft aus einem mittels dünnem Blechplättchen eingefaßten Prisma,einem Prismaträger,dem Träger des Objektivs, dem Objektiv,einer zur Hinterleuchtung des Prisma angeordneten Beleuchtungseinrichtung , Haltewinkeln,dem Kameraelement sowie der Streuscheibe.

Dabei hat es sich besonders vorteilhaft erwiesen,Leuchtdioden rot zur Hinterleuchtung einzusetzen sowie als Material für die Streuscheibe ein lichtdurchlässiges ,streuendes Material einzusetzen.Erfindungsgemäß von besonderem Vorteil ist der Einsatz von Makrolon,welches die Färbung des Lichts ausreichend streut.

Mittels dem das Prisma einfassenden Blechplättchen wird ein Störfeld erzeugt ,welches bei Fingerauflage eine Feldstörung bewirkt ,die zur Signalauswertung führt und dem Lebendtest dient.Erfindungsgemäß wird weitergehend vorgeschlagen, über eine Infrarotmeßeinrichtung,die vorteilhaft als Bauelement hinter der Auflagefläche mit Reflexkörper zum reflektieren des Signals ausgebildet ist.Weiterführend vorgeschlagen wird eine auf der Grundlage einer optischen Sensorik beruhenden Meßeinrichtung,die anhand der Bewegung des Blutes den Sauerstoffgehalt desselben feststellen kann und somit den Lebendtest erfüllt und das Ergebnis an die Speicher bzw. Steuereinheit abgibt.

Figur 3 stellt den optischen Aufbau schematisch dar.

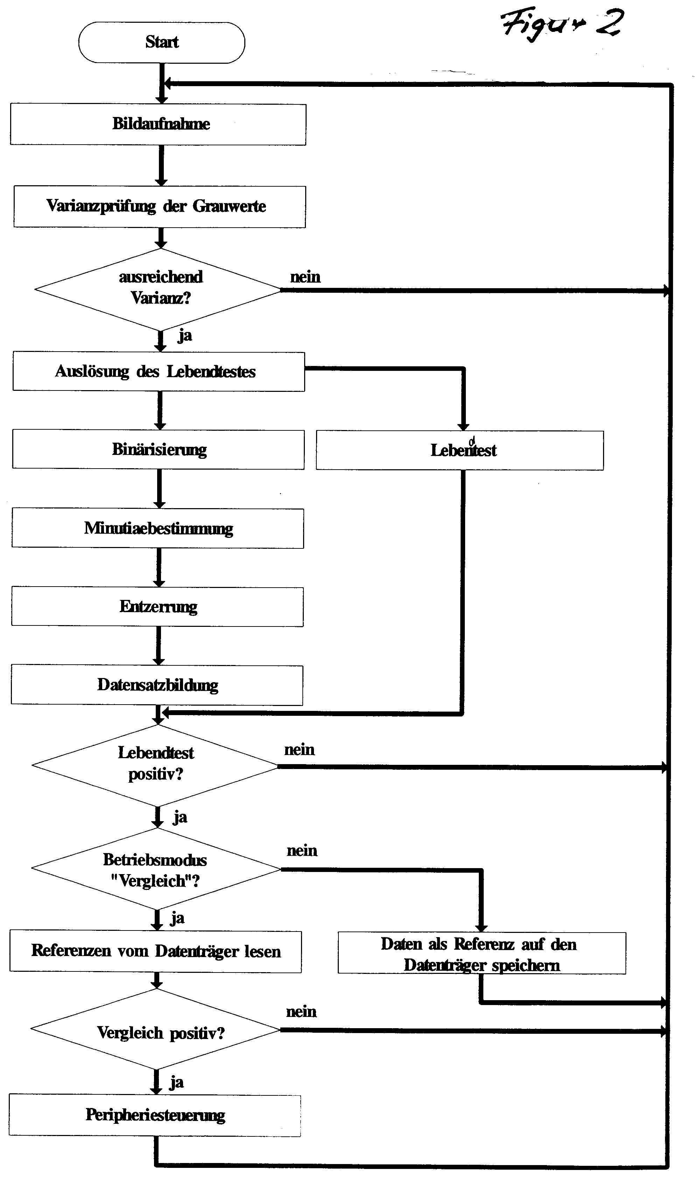

Figur 2 gibt den Verfahrensablauf an.

In Figur 1 ist das Verfahrensschema dargestellt.

Der CCD-Sensor(9) nimmt stetig Bilder auf und sendet ein VIDEOSIGNAL an den A/D-Wandler(2) sowie die Bildspeichersteuerung(4).

Der A/D-Wandler(2) wandelt die analogen Videosignale in digitale Bildinformationen, sogenannte Grauwerte um, welche zum Bildspeicher(3) gesendet werden.

Das PICTURE-RECORD-Signal ist von der Bildverarbeitungseinheit(5) gesetzt, damit wird von der Bildspeichersteuerung(4) das WRITE-ENABLE-Signal gesetzt.

Der Bildspeicher(3) kann also die vom A/D-Wandler(2) empfangenen Grauwerte speichern.

Ist ein Vollbild vollständig aufgenommen und in den Bildspeicher(3) geschrieben, sendet die Bildspeichersteuerung(4) an die Bildverarbeitungseinheit(5) ein PICTURE-READY-Signal.

Damit wird der Bildverarbeitungseinheit(5) mitgeteilt, daß ein Bild zur Auswertung und Bearbeitung zur Verfügung steht.

Von der Bildverarbeitungseinheit(5) wird an verschiedenen Bereichen des aufgenommenen Bildes die Grauwertvarianz geprüft.

Liegt kein Finger auf dem Prisma auf, ist die Varianz der Grauwerte gering.

Bei Auflage eines Fingers auf dem Prisma erhöht sich die Varianz der Grauwerte.

Von Vorteil ist, daß nur bestimmte Bereiche des Bildes zur Grauwertvarianzbestimmung ausgewertet werden ,nicht das gesamte Bild. Damit wird ein hoher Rechenaufwand vermieden.

Es werden solange Bilder aufgenommen, bis die Grauwertvarianz einen bestimmten vorzugebenden Schwellwert überschreitet.

Dies wird erreicht, wenn ein Finger auf dem Prisma aufliegt und ein kontrastreiches Bild liefert.

Damit wird gewährleistet, daß nur Bilder mit ausreichendem Kontrast weiter bearbeitet werden. Die Bildverarbeitungseinheit(5) wird nicht damit beschäftigt, Bilder ungenügender Qualität auszuwerten und zu bearbeiten.

Die Abweisungsrate wird erheblich gesenkt.

Wurde der Schwellwert der Grauwertvarianz überschritten, wird von der Bildverarbeitungseinheit(5) das PICTURE-RECORD-Signal gelöscht. Die Bildspeichersteuerung(4) löscht das WRITE-ENABLE-Signal.

Es wird kein neues Bild in den Bildspeicher (3) geschrieben.

Das aufgenommene Bild steht nun zur weiteren Verarbeitung zur Verfügung.

Von der Bildverarbeitungseinheit(5) wird das Lebendtestauslösesignal gesetzt und die Lebendtesteinheit(6) zum Start veranlaßt. Mit Hilfe des Lebendtestes soll verhindert werden, daß anstelle eines echten lebenden Fingers Imitationen z.B. in Form von Silikonattrappen bzw. abgetrennten Fingern zur Täuschung des Systems eingesetzt werden können.

Das aufgenommene Bild wird binärisiert. Dadurch wird eine drastische Verringerung der Bildinformationsmenge und somit eine kürzere Rechenzeit erreicht.

Die Binärisierung erfolgt mit dynamischen Schwellwert.

Die Binärisierschwelle wird nicht für das gesamte Bild verwendet, weil dann im Binärbild Qualitätsverluste auftreten, die auf eine ungleichmäßige Beleuchtung des Fingers zurückzuführen sind.

Das Bild wird in kleine Quadranten unterteilt, für die jeweils eine eigene Binärisierschwelle errechnet wird.

Besonders vorteilhaft ist, daß dadurch Qualitätsschwankungen in der Beleuchtung nur zu einem geringen Anteil Einfluß auf die Qualität des Binärbildes haben.

Ein weiterer Vorteil ist, daß auch Teile des Bildes mit hoher Qualität binärisiert werden können, die nur wenig Kontrast aufweisen.

Im Binärbild werden die Minutiae (Linienenden, Linienverzweigungen, etc) sowie der Kernpunkt bestimmt.

Zur Identifizierung der Minutiae als solche wird deren Form ausgewertet.

Dazu werden im Binärbild nur die Kanten, also nur die Übergänge zwischen hellen und dunklen Pixeln benötigt.

Dabei ist von Vorteil, daß zur Minutiaebestimmung nicht die gesamte Bildinformation benötigt wird, sondern nur ein geringer Teil. Damit wird der Rechenaufwand erheblich reduziert. Das Bild kann schneller zur Minutiaefindung durchlaufen werden.

Die Positionen der identifizierten Minutiae sowie Ihre Art werden festgehalten.

Ein weiterer Vorteil ist, daß nicht nur Positionen bzw. Abstände der Minutiae untereinander sondern auch Informationen über ihre Art zur Identifizierung des Fingers, welche nachfolgend beschrieben wird, verwendet werden. Damit wird ein höherer Identifizierungsgrad erreicht.

Das Binärbild ist, da das Grauwertbild über ein Prisma aufgenommen wurde, verzerrt. Dies birgt die Gefahr in sich, daß je nach Position der Auflage des Finger auf dem Prisma bei gleichen Fingern, andere Relationen zwischen den Minutiaen auftreten.

Um diese Gefahr zu eliminieren, wird eine Entzerrung durchgeführt. Die Entzerrung bezieht sich auf Grund der hohen Rechenzeit nicht auf das Gesamtbild, sondern nur auf die Koordinaten der ermittelten Minutiae. Somit kann ein Finger beliebig gedreht auf dem Prisma positioniert werden, ohne das dies einen Einfluß auf die Relationen zwischen den einzelnen Minutiaen hat.

Die Lebendtesteinheit(6) liefert das Ergebnis des Lebendtestes.

Ist der Lebendtest positiv, können die gewonnenen Daten je nach Betriebsmodi als Referenz gespeichert oder aber mit vorhandenen Referenzen verglichen werden.

Im Bertriebsmodi "Anlernen" werden die Daten als Referenz auf Datenträgern (z.B. Festplatte, Chipkarte) gespeichert.

Zur Speicherung von Referenzen muß eine anzulernende Person autorisiert werden, um zu verhindern, daß unerwünschte Referenzen die Sicherheit des Systems untergraben. Bei uns erfolgt das durch einen Masterkey, der nur autorisierten Personen ein Anlernen am System erlaubt. Der Masterkey kann nicht dubliziert werden. Der Code auf dem Masterkey wird bei dessen Benutzung per Zufallsgenerator geändert und ist zusätzlich verschlüsselt.

Auch die Art der Verschlüsselung wird bei jeder Benutzung geändert.

Im Betriebsmodus "Vergleichen" können die aus dem Fingerabdruckbild ermittelten Daten mit vorher gespeicherten Referenzen verglichen werden. Im Falle eines positiven Vergleichsergebnisses können periphere Geräte (z.B. Türöffner etc. ) geschaltet, oder Zugriffe auf Daten (z.B. Datenbanken) erlaubt werden.

Bei negativem Vergleichsergebnis wird die Prüfperson abgewiesen.

Nach Beendigung des Vergleichs- oder Anlernvorganges bzw. nach negativem Lebendtestergebnis wird von der Bildverarbeitungseinheit(5) das PICTURE-RECORD-Signal und somit durch die Bildspeichersteuerung(4) das WRITE-ENABLE-Signal gesetzt.

In den Bildspeicher(3) können nun wieder die vom A/D-Wandler(2) digitalisierten Bildinformationen geschrieben und somit erneut Fingerabdruckbilder aufgenommen werden.

## Patentansprüche

1. Verfahren zur Mustererkennung, insbesondere Fingerabdruck-Identifikation durch Auflage des Musters auf eine Bildaufnahmevorrichtung zur optischen Aufbereitung des zu erkennenden Musters, Erzeugung eines Aufnahmestartsignals bei Auflage des Musters, Bildaufnahmevorrichtung mit Prisma, Objektiv mit optischem Sensor, insbesondere eines CCD-Sensors zur Wandlung der Bildinformationen in analoge, elektrische Signale, Umwandlung der analogen, elektrischen Signale in digitale Grauwertinformationen, Speicherung und mathematische Bewertung der digitalen Grauwertinformationen sowie Speicherung der Bewertungsergebnisse und Vergleich dieser Informationen mit entsprechenden Referenzinformationen in einer elektronischen Verarbeitungseinheit und Auswertung des Vergleiches hinsichtlich Übereinstimmung sowie Erzeugung eines vom Vergleichsergebnis abhängigen elektrischen Signals gekennzeichnet dadurch, daß ein fehlertoleranter Mustervergleich insbesondere durch unterschiedliche Positionierung des Musters auf der Bildaufnahmevorrichtung derart erfolgt, daß in der Ebene des CCD-Sensors ein gleichmäßig scharfes Bild erzeugt wird durch Kippen des CCD-Elementes unter Einhaltung der Scheimpflug-Bedingung und die dadurch entstehende optische Verzerrung des Bildes in der elektronischen Verarbeitungseinheit nach der Wandlung in digitale Grauwertinformationen mathematisch aufgehoben wird, zur optimalen Fälschungssicherheit das Aufnahmestartsignal aus einer UND-verknüpften Abfrage eines Signals (S1), erzeugt durch die Erkennung der Auflage des Musters auf die Bildaufnahmevorrichtung, mit mindestens einem Signal (S2) zur erfolgreichen Lebenderkennung, erzeugt durch eine Elektronikeinheit zur Bewertung bestimmter relevanter Lebensfunktionen, z. B. eines Fingers, und mindestens einem Signal (S3), erzeugt durch eine Elektronikeinheit zur Bewertung mindestens eines Parameters des elektrischen Bildsignals des CCD-Sensors erfolgt sowie eine begrenzte Zahl n als Abfragewiederholung für die mindestens 3 Signale (S1) bis (S3) existiert, falls ein oder mehrere Signale keine erfolgreiche Bewertung signalisieren und nach Überschreitung der Zahl n eine Sperrung der Bildaufnahmefunktion durch die elektronische Verarbeitungseinheit und deren optische und/oder akustische Signalisation erfolgt.

2. Verfahren zur Mustererkennung, insbesondere Fingerabdruck-Identifikation nach Anspruch 1, gekennzeichnet dadurch, daß die Signale (S1) bis (S3) in analoger Form vorliegen und eine gemeinsame unscharfe Bewertung über einen Fuzzy-Algorithmus derart erfolgt, daß nur bei Erreichung bestimmter, über die elektronische Verarbeitungseinheit vorgebbarer Mindestanforderungen die Freigabe eines Aufnahmestartsignals erfolgt.

3. Anordnung zur Mustererkennung, insbesondere Fingerabdruck-Identifikation, aus Bildaufnahmevorrichtung (1) zur optischen Aufbereitung des zu erkennenden Musters sowie zur Wandlung der optischen Bildinformation in analoge, elektrische Bildsignale mittels optischem Sensor (9), insbesondere CCD-Sensor, Umwandlung der analogen, elektrischen Bildsignale in digitale Bildsignale mittels Analog-Digital-Wandler (3), Speicherung dieser Signale in Halbleiterspeichern (3a), elektronische Verarbeitungseinheit (4) mit Speicher (4a) zur Bewertung der digitalen Bildinformation mit Referenzinformationen aus dem Speicher (4a) mittels installierter Software sowie Bewertung des Erkennungsprozesses mit Signalausgabeeinheit (5) gekennzeichnet dadurch, daß die Bildaufnahmeeinheit (2) besteht aus einem Dreikantenprisma (7), bei dem die Prismenfläche (F3) als Auflage für das zu erkennende Muster dient, einer Lichtquelle (6) deren Lichtstrahlen durch die Fläche (F1) in das Dreikantenprisma (7) eintreten, einer Objektivlinse (8), welche die aus der Fläche (F1) austretenden und von der Prismenfläche (F3) reflektieren und parallel zur optischen Achse (OA) verlaufenden Lichtstrahlen bündelt und das Musterbild in der Abbildungsebene (BE) der dahinter angeordneten CCD-Matrix (9) scharf abbildet, wobei die CCD-Matrix (9) mit ihrer Abbildungsebene (BE) nicht senkrecht zur optischen Achse (OA) des optischen Systems steht. sondern unter Einhaltung der Scheimpflug-Bedingung im Winkel delta < 90°, einer Auflagesensorik (1) zur Erkennung der Auflage des Musters auf die Fläche (F3) des Dreikantenprismas (7) und einer Sensorik (15) zur Lebenderkennung des aufgelegten Musters mit Elektronikeinheit (14) zu deren Signalbewertung und daß die Fläche (F3) des Dreikantenprismas (7) in einem Winkel alpha = 25° bis 44° zur optischen Achse (OA) des optischen Systems angeordnet ist.

4. Anordnung nach Anspruch 3 ,wobei die Fingerauflage im Winkel von 15 Grad zur Gehäuseoberfläche angeordnet ist.

5. Aufnahmemodul bestehend aus Prisma mit Prismaträger,Objektiv mit Objektivträger,Beleuchtungseinrichtung,Haltewinkeln,dem Kameraelelement sowie der Streuscheibe dadurch gekennzeichnet,daß Leuchtdioden rot zur Hinterleuchtung eingesetzt werden.

6. Aufnahmemodul nach Anspruch 5 ,wobei die Streuscheibe aus einem lichtdurchlässigem ,die Färbung des Lichts streuenden Material besteht.

Fig. 1

EP 0 640 933 A2

Figur 2

```
                    ┌──────────────┐
                    │    Start     │
                    └──────┬───────┘
                           │
        ┌──────────────────┴──────────────────┐
        │            Bildaufnahme              │
        └──────────────────┬──────────────────┘
                           │
        ┌──────────────────┴──────────────────┐
        │    Varianzprüfung der Grauwerte      │
        └──────────────────┬──────────────────┘
                           │
                    ◇ ausreichend ◇──── nein ──────►
                    ◇  Varianz?   ◇
                           │ ja
        ┌──────────────────┴──────────────────┐
        │    Auslösung  des  Lebendtestes      │───────┐
        └──────────────────┬──────────────────┘       │
                           │                           │
        ┌──────────────────┴──────────────────┐   ┌────┴─────────┐
        │            Binärisierung             │   │  Lebentest   │
        └──────────────────┬──────────────────┘   └──────────────┘
                           │
        ┌──────────────────┴──────────────────┐
        │         Minutiaebestimmung           │
        └──────────────────┬──────────────────┘
                           │
        ┌──────────────────┴──────────────────┐
        │             Entzerrung               │
        └──────────────────┬──────────────────┘
                           │
        ┌──────────────────┴──────────────────┐
        │         Datensatzbildung             │
        └──────────────────┬──────────────────┘
                           │
                    ◇  Lebendtest  ◇──── nein ──────►
                    ◇   positiv?    ◇
                           │ ja
                    ◇ Betriebsmodus ◇─── nein ───┐
                    ◇  "Vergleich"? ◇             │
                           │ ja                   │
        ┌──────────────────┴──────────────┐   ┌───┴────────────────────────┐
        │ Referenzen vom Datenträger lesen │   │ Daten als Referenz auf den │
        └──────────────────┬───────────────┘  │   Datenträger speichern     │
                           │                   └───────────┬────────────────┘
                    ◇ Vergleich positiv? ◇──── nein ──────►
                           │ ja
        ┌──────────────────┴──────────────────┐
        │         Peripheriesteuerung          │
        └──────────────────────────────────────┘
```

7

F3

F2

F1

10

8

9

Fig. 3:

7

OE

HH'

BE

a

a'

9

α

δ

δ'

8